# FASCICULE DE BREVET EUROPEEN

(11) **EP 0 847 268 B1**
(45) Date de publication et mention de la délivrance du brevet: **12.09.2001**
(21) Numéro de dépôt: 96929383.6
(22) Date de dépôt: 29.08.1996
(51) Int. Cl.: A61K 7/00, A23L 1/226, A24B 15/34

(54) **COMPOSITIONS RAFRAICHISSANTES**
ERFRISCHENDE ZUSAMMENSETZUNGEN
REFRESHING COMPOSITIONS

(30) Priorité: 29.08.1995 US 520399; 09.04.1996 US 629598; 21.08.1996 US 701141
(43) Date de publication de la demande: 17.06.1998
(73) Titulaire: V. Mane Fils S.A., 06620 Le Bar-sur-Loup (FR)
(72) Inventeur: MANE, Jean, 06130 Grasse (FR); PONGE, Jean-Louis, 06130 Grasse (FR)
(74) Mandataire: Boulinguiez, Didier
(86) Numéro de dépôt international: FR9601333
(87) Numéro de publication internationale: WO9707771

(56) Documents cités:
- WO-A-93/23005
- WO-A-93/25177
- DE-A- 2 608 226
- US-A- 3 111 127
- US-A- 4 157 384

## Description

La présente invention se rapporte à des compositions rafraîchissantes ayant une perception de fraîcheur unique qui procure à l'utilisateur un effet de rafraîchissement plaisant sans aucune marque d'amertume. L'invention se rapporte également à des produits contenant un ou plusieurs composés rafraîchissants.

Plusieurs composés sont connus pour fournir une sensation de fraîcheur lorsqu'ils sont ingérés ou mis en contact avec le corps. Il est évident que le menthol agit sur les récepteurs de froid aux extrêmités nerveuses afin de provoquer cet effet de fraîcheur. Etant donné que le menthol présente une forte odeur de menthe et une grande volatilité relative, plusieurs autres composés rafraîchissants furent développés et cités dans la littérature technique comme aromatisants ou odorants dans diverses compositions topiques et ingestibles. Par exemple, le brevet américain n° 5,009,893 propose l'utilisation du menthol en combinaison avec des composés N-substitué-p-menthane carboxamide comme compositions rafraîchissantes dans les produits comestibles.

La demande de brevet internationale n° WO93/23005 propose des compositions rafraîchissantes pour produits topiques ou comestibles qui comprennent un cétal et un rafraîchissant secondaire qui peut être choisi parmi le menthol, les carboxamides et leurs mélanges. Plusieurs autres références y sont mentionnées qui décrivent des composés ayant une saveur ou un arôme semblables au menthol, incluant le menthyl carbinol, les saccharides esters de menthol et plusieurs amides. De même le 2,3-p-menthane diol a été décrit comme ayant un goût rafraîchissant aigu.

La demande de brevet allemand 2 339 661 décrit des compositions aromatiques qui incluent le menthol ou des esters de menthol et d'acides carboxyliques hétérocycliques. L'ester préféré est l'ester de l'acide menthyl-2-pyrrolidone-5-carboxylique.

La demande de brevet allemand 26 08 226 décrit une composition qui présente un effet rafraîchissant physiologique. Les composés rafraîchissants décrits incluent des esters de menthol et d'acides hydroxycarboxyliques naturels ayant de 2 à 6 atomes de carbone qui sont estérifiés avec un groupe alkyle en C1-C4. L'acétate de menthyle et le lactate de menthyle constituent les composés rafraîchissants préférés de ce document. Enfin, un autre composé rafraîchissant commercialement disponible est le 3-menthoxypropane-1,2 diol.

Par conséquent, plusieurs composés sont connus qui procurent des propriétés rafraîchissantes (ou réfrigérantes ou refroidissantes) et qui sont utiles dans une large gamme de produits. Cependant, il existe encore un besoin de disposer de compositions rafraîchissantes ayant un effet rafraîchissant et/ou une perception de goût améliorés. Ainsi, un objet de la présente invention est de fournir des compositions rafraîchissantes améliorées.

Un autre objet de la présente invention est de fournir des compositions rafraîchissantes ayant une sensation rafraîchissante et une perception de goût uniques.

Un autre objet de la présente invention est de fournir des compositions rafraîchissantes incluant deux ou plus agents rafraîchissants qui procurent une sensation rafraîchissante et une perception de goût complémentaires.

Un autre objet de la présente invention est de fournir une nouvelle composition rafraîchissante qui utilise un rafraîchissant non-toxique ayant une sensation de fraîcheur unique et une perception de goût unique.

Selon un premier aspect, la présente invention se rapporte à une composition sélectionnée parmi les produits topiques, les produits de soin oral, les produits de soin nasal, les produits de toilette, les produits ingestibles, les chewing-gums et les produits à fumer, le tabac à mâcher, les chiques et les produits de tabac à priser et à chiquer, qui incluent un produit de base et une quantité efficace d'un composé rafraîchissant choisi parmi le monomenthyl succinate, les sels de métaux alcalins du monomenthyl succinate et les sels de métaux alcalino-terreux du monomenthyl succinate.

Selon un deuxième aspect, la présente invention se rapporte à une composition incluant un rafraîchissant choisi parmi le monomenthyl succinate, les sels de métaux alcalins du monomenthyl succinate et les sels de métaux alcalino-terreux du monomenthyl succinate formulés avec un diluant choisi parmi les aromatisants, les sirops sucrants ou édulcorants, les sirops aromatisants, les huiles aromatisantes et les huiles d'herbes.

La présente invention concerne également des compositions rafraîchissantes qui contiennent un rafraîchissant primaire sélectionné parmi le monomenthyl succinate, les sels de métaux alcalins de monomenthyl succinate et les sels de métaux alcalino-terreux de monomenthyl succinate, et au moins un composant rafraîchissant secondaire.

Le monomenthyl succinate est un composé connu ayant la référence Chemical Abstracts n° 77341-67-4. Il a été utilisé, par exemple, dans le tabac à fumer tel que décrit dans le brevet américain n° 3,111,127. En particulier, ce brevet décrit un tabac à fumer qui incorpore un monoester de menthol synthétique ou naturel et d'un acide polycarboxylique aromatique ou aliphatique saturé ou insaturé ou d'un analogue substitué d'un tel acide. Ces produits de tabac furent évalués et observés comme brûlant de façon plus lente à feu latent qu'un produit témoin comparable, comme ayant une fermeté accrue, et comme nécessitant plus de bouffées sous un régime de fumée contrôlée. En outre, il s'est avéré que lorsqu'ils étaient testés du point de vue organoleptique, ces produits produisaient une fumée ayant un goût plaisant et rafraîchissant et la caractéristique aromatique du menthol. Parmi les esters d'acides polycarboxyliques sont mentionnés l'ester d'acide méthylsuccinique, le mono menthylsuccinate, le monomenthyl-α-α-diméthylsuccinate et le mono mentholmenthylsuccinate.

L'article intitulé "A molecular Approach to Flavor Synthesis. I .Menthol Esters of Varying Size and Polarity" Jabloner, H et Dunbar, B. I., J. of Polymer Science, vol. 18, page 2933-40 (1980) décrit une méthode pour la synthèse du monomenthyl succinate ainsi que du monomenthyl succinate de sodium et d'autres esters de menthol dérivés du monomenthyl succinate. Des solutions à 5 % en poids de plusieurs de ces esters de menthol dans des huiles minérales ou dans l'eau ont été testés par un jury de neuf dégustateurs. 5 % du dimenthyl succinate dans l'huile minérale a été jugé inodore et sans goût, 5 % du monomenthyl succinate de sodium dans l'eau a été trouvé exécrable et amer, et le monomenthyl succinate lui-même n'a pas été goûté.

Il a été trouvé que, de façon surprenante et inattendue, le monomenthyl succinate, les sels de métaux alcalins du monomenthyl succinate et les sels de métaux alcalino-terreux du monomenthyl succinate utilisés dans une gamme de produits ingestibles et topiques à de basses concentrations allant jusqu'à 1 % en poids, donnaient un effet rafraîchissant de longue durée, plaisant, plutôt que le goût amer et exécrable observé par le jury de dégustateurs dans l'article de Jabloner.

La présente invention concerne une composition finie choisie parmi les produits topiques, les produits de soin oral, les produits de soin nasal, les produits de toilette, les produits ingestibles, les chewing-gums, le tabac à mâcher, les chiques et les produits de tabac à priser et à chiquer, et les produits de tabac à fumer qui incluent un produit de base et une quantité efficace de composés rafraîchissants sélectionnés parmi le monomenthyl succinate, les sels de métaux alcalins du monomenthyl succinate et les sels de métaux alcalino-terreux du monomenthyl succinate (ci-après désignés collectivement par le terme "composés rafraîchissants à base de succinate").

De préférence, le rafraîchissant utilisé dans les compositions de la présente invention est sélectionné parmi le monomenthyl succinate, le monomenthyl succinate de sodium, le monomenthyl succinate de potassium, le monomenthyl succinate de lithium, le monomenthyl succinate de calcium, le monomenthyl succinate de magnésium et le monomenthyl succinate de baryum, ainsi que leurs mélanges.

Il a été trouvé que de façon surprenante ces composés rafraîchissants à base de succinate, quand ils sont utilisés à des concentrations basses allant jusqu'à 1 % en poids par rapport au poids total de la composition finie dans laquelle ils sont incorporés, procurent un effet rafraîchissant plaisant et/ou de longue durée sans l'amertume qui aurait dû être observée si l'on en croit l'art antérieur. De plus, à des concentrations allant jusqu'à 1 % en poids, les composés rafraîchissants à base de succinate ne développent pas un goût accentué de menthe dans la bouche ou dans la gorge comme le font les autres rafraîchissants tels que le menthol.

Les composés rafraîchissants à base de succinate selon la présente invention procurent également, quand ils sont ingérés, un effet rafraîchissant dans une région différente de la bouche ou de la gorge que, par exemple, le menthol ou les composés rafraîchissants à base de carboxamide. Il en résulte que les composés rafraîchissants à base de succinate selon la présente invention fournissent un effet rafraîchissant complémentaire ou synergique quand ils sont combinés avec au moins un composé rafraîchissant secondaire.

En outre, les composés rafraîchissants à base de succinate conformes à la présente invention augmentent la sensation gustative de l'alcool dans les boissons alcooliques. De ce fait, les boissons alcooliques comprenant les composés rafraîchissants à base de succinate selon la présente invention sont perçus comme s'ils avaient un contenu en alcool plus élevé que les boissons alcooliques aussi fortes ne comportant pas le composé rafraîchissant à base de succinate. Le choix du composé rafraîchissant à base de succinate pour une utilisation dans une composition rafraîchissante dépendra, dans une certaine mesure, des caractéristiques de solubilité qui sont désirées. Par exemple, le monomenthyl succinate est à peine soluble dans l'eau et plus soluble dans l'huile. Par conséquent, le monomenthyl succinate est particulièrement adapté dans des environnements où la solubilité dans l'huile est avantageuse, bien que le monomenthyl succinate puisse être utilisé dans des environnements aqueux si une concentration inférieure à la limite de solubilité dans l'eau est utilisée. Les sels de métaux alcalins et alcalino-terreux du monomenthyl succinate sont substantiellement plus solubles dans l'eau et, de ce fait, sont plus utiles dans des produits où la solubilité dans l'eau est avantageuse.

Les compositions finies dans lesquelles les composés rafraîchissants à base de succinate peuvent être utilisés incluent une grande variété de compositions ingestibles et de compositions topiques pour application au corps humain ou animal. Les compositions ingestibles comprennent les aliments pour consommation humaine ou animale, les boissons et les autres compositions ingérées oralement pour humains ou animaux telles que les médicaments, les produits antacides et les laxatifs, de même que les compositions de chewing-gums. Les compositions topiques comprennent une grande variété de compositions pour application aux hommes ou aux animaux telles que les articles de toilette, les produits de soins oraux, les produits de soin nasal, les lotions, les huiles et les pommades. Les produits de tabac comprennent le tabac à mâcher, les chiques, le tabac à priser, ainsi que les filtres, les papiers combustibles et les feuilles d'enrobage utilisées dans les produits de tabac à fumer.

En formulant les compositions de la présente invention, le composé rafraîchissant peut être utilisé sous la forme d'une composition rafraîchissante, d'une composition aromatisante et/ou le composé rafraîchissant, la composition rafraîchissante ou la composition aromatisante peuvent être incorporés dans un matériau support qui peut être inerte ou qui peut contenir d'autres ingrédients actifs de la composition finie. Une grande variété de matériaux supports peut être utilisée comprenant, par exemple, des solvants polaires, des huiles, des graisses, des solides finement divisés, des maltodextrines, des cyclodextrines, des gommes, des résines naturelles ou synthétiques et n'importe quel autre matériau support connu pour compositions rafraîchissantes ou aromatisantes.

Les compositions ingestibles conformes à la présente invention comprennent, sans qu'il n'y ait de limitation, les boissons alcooliques et non alcooliques, les compositions de confiserie incluant les comprimés de confiserie, les bonbons durs au sucre cuit, les chewing-gums, les bonbons à base de pectine, les bonbons à mâcher, les bonbons fourrés à la crème et les fondants, les boissons carbonatées, les mélanges en poudre pour boissons, les boissons distillées, les boissons alcooliques, les boissons non alcooliques, les eaux minérales, les produits de boulangerie, les produits laitiers, les glaces aux fruits, les confitures, les gelées, les produits à base de gélatine, les puddings et les produits alimentaires pour animaux.

Les bonbons sans sucre constituent un autre type de composition ingestible conforme à la présente invention. Les bonbons sans sucre ont typiquement des points d'ébullition plus élevés que les bonbons correspondants à base de sucre et donc tout produit aromatisant qui leur est ajouté doit être stable à des températures plus élevées de façon à pouvoir être ajouté avec succès au bonbons sans sucre. Les bonbons sans sucre peuvent inclure les bonbons durs au sucre cuit, les bonbons à base de pectine, les bonbons à mâcher et les bonbons fourrés. De plus, les chewing-gums sans sucre peuvent être fabriqués conformément à la présente invention. La quantité de composition rafraîchissante à incorporer dans les bonbons sans sucre et/ou les gommes sans sucre est la même que celle qui serait utilisée pour les bonbons ou les gommes correspondantes à base de sucre.

Les compositions topiques de la présente invention incluent, notamment, les articles de toilette tels que les crèmes pour visage, les poudres de talc, les huiles pour cheveux, les shampoings, les sels et huiles de bain, les savons de toilette, les eaux de Cologne, les antitranspirants, les eaux de toilette, les parfums, les lotions et les crèmes de rasage, les savons, les crèmes, les dentifrices, les bains de bouche, les lotions capillaires et autres produits similaires.

Les composés rafraîchissants à base de succinate selon la présente invention peuvent également être incorporés dans des médicaments ingestibles ou topiques tels que les produits contre la toux, les antacides, les pastilles, les anti-irritants, les pommades, les lotions, les produits analgésiques oraux et les autres produits similaires. En outre, d'autres compositions telles que les adhésifs pour une gamme d'utilisation où la perception de goût est importante peuvent incorporer des compositions rafraîchissantes et aromatisantes conformes à la présente invention.

Les composés rafraîchissants à base de succinate sont incorporés dans divers produits du tabac en conformité avec la présente invention. Selon une première réalisation, les composés rafraîchissants à base de succinate sont incorporés dans le tabac à mâcher, les chiques ou le tabac à chiquer. Selon une autre réalisation, les composés rafraîchissants à base de succinate sont associés avec les produits de tabac à fumer soit en incorporant les composés rafraîchissants à base de succinate dans le papier combustible, la feuille d'enrobage ou le filtre des produits de tabac à fumer ou en appliquant les compositions rafraîchissantes à base de succinate à la surface du papier combustible de la feuille d'enrobage et/ou du filtre des produits de tabac à fumer.

Par produits de tabac, on entend les produits tels que les cigarettes, les cigares, le tabac à mâcher, les chiques, le tabac à chiquer, le tabac à priser, qui incluent comme composant le tabac ou un produit dérivé du tabac. Par exemple les cigarettes à la girofle et les cigares ainsi que les tabacs à mâcher aromatisés et les produits analogues sont inclus dans le domaine des produits de tabac dans le cadre de la présente invention. La chique est une forme de tabac à chiquer qui est placée dans la bouche, soit sous forme d'une boulette faite à la main, soit sous la forme de produit contenu dans un sachet de papier à haute porosité.

Les composés rafraîchissants à base de succinate selon la présente invention procurent un arrière-goût rafraîchissant quand ils sont utilisés dans certains produits de tabac. En outre, quand ils sont utilisés en combinaison avec un composé tel que le menthol, les composés rafraîchissants à base de succinate procurent un effet rafraîchissant complémentaire sans augmenter de façon indésirable le goût de menthe du menthol.

Dans la confection des produits de tabac de la présente invention, le composé rafraîchissant peut être utilisé sous la forme d'une composition rafraîchissante ou le composé rafraîchissant ou la composition peuvent être incorporés dans un matériau support qui peut être inerte ou qui peut contenir d'autre ingrédients actifs de la composition finie. Une grande variété de matériaux supports peut être employée incluant, par exemple, les solvants polaires, les huiles, les graisses, les solides finement divisés, les maltodextrines, les cyclodextrines, les gommes, les résines et n'importe quel autre matériau support connu pour de telles compositions.

La quantité de composition rafraîchissante incorporée dans chacun des produits de tabac de la présente invention variera selon le composé particulier, le degré d'effet rafraîchissant voulu et la force des autres rafraîchissants dans la composition. Typiquement, le composé rafraîchissant à base de succinate représentera de 0,01 à 0,5 % en poids du produit de tabac. De préférence, le composé rafraîchissant à base de succinate représentera de 0,02 à 0,3 % en poids par rapport au poids total du produit de tabac.

Les composés rafraîchissants à base de succinate sont appliqués directement au tabac de n'importe quelle manière conventionnelle telle que pulvérisation, brossage ou injection, par exemple. Quand ils sont appliqués au papier combustible, à la feuille d'enrobage ou au filtre des produits de tabac à fumer, les composés rafraîchissants à base de succinate peuvent être pulvérisés ou déposés à la brosse, ou, dans le cas des filtres, peuvent être injectés dans les filtres. N'importe quel autre moyen adéquat peut être utilisé.

La présente invention concerne également les produits de tabac incluant une combinaison d'un agent rafraîchissant primaire choisi parmi le monomenthyl succinate, les sels de métaux alcalins du monomenthyl succinate, les sels de métaux alcalino-terreux du monomenthyl succinate et leurs mélanges, avec au moins un composé rafraîchissant secondaire. Généralement, la teneur en composés rafraîchissants secondaires dans les produits de tabac selon la présente invention est d'environ 10 % en poids à environ 700 % en poids, de préférence d'environ 20 % en poids à environ 650 % en poids, et plus préférentiellement encore d'environ 30 % en poids à environ 500 % en poids, par rapport au poids d'agent rafraîchissant primaire.

D'autres compositions préférées qui incorporent le rafraîchissant à base de succinate selon la présente invention sont des comprimés de confiserie, des bonbons durs aux sucres cuits, des chewing-gums, des bonbons à mâcher, des bonbons à base de pectine, des bonbons fourrés, des fondants, des pâtes dentifrice, des produits de rinçage pour la bouche, des rafraîchissants d'haleine, des boissons alcooliques et non alcooliques, des boissons carbonatées et des mélanges secs pour boissons.

La quantité de composition rafraîchissante incorporée dans chacune de ces compositions finies variera selon le composé particulier, le degré d'effet rafraîchissant désiré et la force des autres aromatisants dans la composition. Généralement, le composé rafraîchissant à base de succinate représentera de 0,001 à 1,0% en poids de la composition finie. De préférence, le composé rafraîchissant à base de succinate représentera de 0,005 à 0,5% en poids, par rapport au poids total de la composition finie.

Selon un second aspect, la présente invention concerne une composition aromatisante comprenant de 1 à 80% en poids d'un agent rafraîchissant choisi dans le groupe du monomenthyl succinate, des sels de métaux alcalins du monomenthyl succinate, des sels alcalino-terreux du monomenthyl succinate et leurs mélanges, et 20 à 99% en poids d'un diluant aromatisant qui comprend au moins un agent aromatisant choisi dans le groupe des arômes de fruits, des huiles d'herbes, des sirops sucrants ou édulcorants, des sirops aromatisants et des huiles aromatisantes. Cette composition est particulièrement adaptée pour une utilisation dans les compositions ou les produits visés par la présente invention.

Les aromatisants peuvent être choisis parmi les arômes ou parfums de fruits tels que l'arôme de fraise, les huiles d'herbes telles que l'huile d'eucalyptus, l'huile de menthe poivrée, l'huile de menthe verte, ou d'autres arômes connus ou d'huiles aromatisantes utilisés de façon conventionnelle dans les compositions ingestibles et les compositions destinées à entrer en contact avec les corps humains ou animaux, incluant des arômes tels que les sirops aromatisants tels que le sirop de sorbitol ou d'autres sirops sucrants ou aromatisants.

Ces compositions aromatisantes peuvent être diluées de façon optionnelle avec un solvant polaire tel que, par exemple, l'alcool éthylique, l'acétate d'éthyle, le propylène glycol, l'alcool isopropylique et la glycérine. Le solvant agit comme matériau support qui aide à incorporer la composition aromatisante dans le produit. Le diluant peut éventuellement comprendre un ou plusieurs composés conventionnels additionnels sélectionnés dans le groupe des colorants, lubrifiants, épaississants, émulsifiants, plastifiants et agents encapsulants tels que les gommes, les amidons, les dextrines et les cyclodextrines.

De façon avantageuse, les compositions aromatisantes comprennent de 1 à 80% en poids d'un agent rafraîchissant choisi dans le groupe du monomenthyl succinate, des sels de métaux alcalins du monomenthyl succinate, des sels alcalino-terreux du monomenthyl succinate et leurs mélanges, et 20 à 99% en poids d'un diluant aromatisant qui comprend au moins un agent aromatisant choisi dans le groupe des arômes de fruits, des sirops sucrants ou édulcorants, des sirops aromatisants et des huiles d'eucalyptus, de menthe poivrée, et de menthe verte.

Les compositions aromatisantes conformes à l'invention peuvent également comprendre de 5 à 80% en poids d'un agent rafraîchissant choisi dans le groupe du monomenthyl succinate, des sels de métaux alcalins du monomenthyl succinate, des sels alcalino-terreux du monomenthyl succinate et leurs mélanges, et 20 à 95% en poids d'un diluant aromatisant qui comprend au moins un agent aromatisant choisi dans le groupe des arômes de fruits, des huiles d'herbes, des sirops sucrants ou édulcorants, des sirops aromatisants et des huiles aromatisantes.

Généralement, la composition aromatisante comprendra de 1 à 80% en poids de composé rafraîchissant à base de succinate et de 20 à 99% en poids de diluant aromatisant et de solvant polaire optionnel. Les compositions aromatisantes préférées comprennent de 5 à 50% en poids de composé rafraîchissant à base de succinate et de 50 à 95% en poids de diluant aromatisant et de solvant polaire optionnel.

La présente invention est également relative à une combinaison d'agents rafraîchissants primaires choisis parmi le monomenthyl succinate, les sels de métaux alcalins du monomenthyl succinate, les sels de métaux alcalins du monomenthyl succinate et leurs mélanges, avec au moins un composé rafraîchissant secondaire.

Les composés rafraîchissants secondaires qui peuvent être utilisés en combinaison avec le produit rafraîchissant primaire de la présente invention incluent le menthol, les carboxamides, les cétals, l'acétate de menthyle, le lactate de menthyle, le 3-menthoxypropane-1,2 diol et leurs mélanges. Les compositions rafraîchissantes à base de carboxamides et de cétals sont connues dans l'art antérieur et peuvent être trouvées, par exemple, dans le brevet US n° 5,009,893 et la demande de brevet internationale n° WO-93/23005, dont les descriptions sont incorporées par référence. Les rafraîchissants secondaires restants sont des agents rafraîchissants connus, dont certains sont disponibles commercialement.

Plus particulièrement, les produits rafraîchissants secondaires à base de carboxamide sont sélectionnés parmi les N-substitué-p-menthane-3-carboxamides de formule : 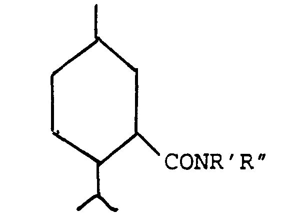 dans laquelle R', quand il est pris séparément, représente l'hydrogène ou un radical aliphatique contenant jusqu'à 25 atomes de carbone ; R", quand il est pris séparément, représente le radical hydroxy ou un radical aliphatique contenant jusqu'à 25 atomes de carbone, à condition que quand R' est l'hydrogène, R" peut également être un radical aryle ayant jusqu'à 10 atomes de carbone, choisi dans les radicaux phényl substitué, phényl alkyle, phényl alkyle substitué, naphtyl, naphtyl substitué et pyrydyl ; et R' et R" quand ils sont pris ensemble avec l'atome d'azote auquel ils sont attachés, représentent un groupe cyclique ou hétérocyclique ayant jusqu'à 25 atomes de carbone ;
les carboxamides acycliques tertiaires et secondaires de formule : 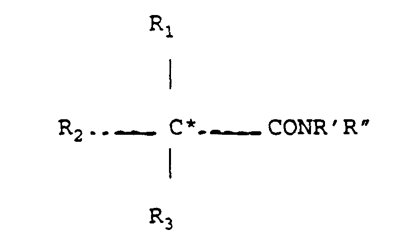 dans laquelle R' et R", quand ils sont pris séparément, sont chacun d'hydrogène, un groupe alkyle en C₁-C₅ ou un hydroxyalkyle en C₁-C₈ et fournissent un total d'au plus 8 atomes de carbone, avec la condition que quand R' est l'hydrogène, R" peut également être un groupe alkycarboxyalkyle ayant jusqu'à 6 atomes de carbone ; R' et R", quand ils sont pris ensemble représentent un groupe alkylène ayant jusqu'à 6 atomes de carbone, les extrémités opposées de ce groupe étant attachées à l'atome d'azote du groupe amide de façon à former un hétérocycle azoté dont la chaîne carbonée peut éventuellement être interrompue par l'oxygène ; R₁ est l'hydrogène ou un groupe alkyle C₁-C₅; et R₂ et R₃ sont chacun un groupe alkyle C₁-C₅ ; avec les conditions que (i) R₁, R₂, et R₃ ensemble fournissent un total d'au moins 5 atomes de carbone, de préférence de 5 à 10 atomes de carbone ; et (ii) quand R₁ est l'hydrogène, R₂ est un groupe alkyle C ₂-C ₅ et R ₃ est un groupe alkyle C₂-C₅ et au moins l'un des groupes R ₂ et R₃ est branché, de préférence dans une position α ou β relativement à l'atome de carbone marqué (*) dans la formule, ainsi que leurs mélanges.

Les compositions rafraîchissantes à base de cétal peuvent être représentées par la formule 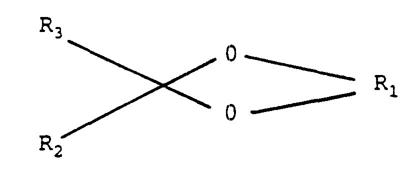 dans laquelle R₁ représente un radical alkylène C₂-C₆ ayant au moins un, mais pas plus de trois groupe(s) hydroxyle, et soit R₂ et R₃ indépendamment l'un de l'autre représentent un groupe alkyle C₁-C₁₀ qui est éventuellement substitué par un à trois radicaux choisis dans le groupe comprenant les groupes hydroxyle, amino et halogène, les cycloalkyle en C₅-C₇, de préférence le cyclohexyle, et un aryle en C₆-C₁₂, de préférence le phényle, avec la condition que le total des atomes de carbone de R₂ et R₃ n'est pas inférieur à 3, où R₂ et R₃ ensemble représentent un radical alkylène qui, ensemble avec l'atome de carbone qui porte les radicaux R₂ et R₃, forme un noyau à 5-7 atomes, éventuellement substitué par des groupes alkyle C₁-C₆.

Les quantités relatives des rafraîchissants primaires et secondaires dans la composition de la présente invention peuvent être variées sur une large gamme de compositions, selon l'arôme particulier voulu. Par exemple, quand le goût de menthe prononcé du menthol est souhaité, une combinaison d'une grande quantité de menthol avec une quantité relativement faible de rafraîchissants à base de succinate conformes à la présente invention peut être souhaitée. D'autres combinaisons potentielles de rafraîchissants primaires avec un ou plusieurs composants rafraîchissants secondaires sont à la portée de l'homme du métier.

Généralement, le taux de composant rafraîchissant secondaire dans la composition rafraîchissante selon la présente invention est d'environ 0,05 % en poids à environ 95 % en poids, de préférence d'environ 0,1 % en poids à environ 70 % en poids, et plus préférentiellement encore d'environ 0,5 % en poids à environ 50 % en poids, par rapport au poids total de la composition. En particulier, les compositions rafraîchissantes sont faites en mélangeant les rafraîchissants primaires et secondaires, de manière conventionnelle.

Certaines compositions selon la présente invention sont illustrées dans les exemples suivants.

### Procédure expérimentale pour les exemples :

Pour chacun des exemples 1 à 10 qui suivent, un panel de dégustation composé de 10 à 12 personnes a goûté les produits. Les dégustateurs avaient été instruits d'avoir à donner des informations sur l'effet rafraîchissant et tout goût désagréable, et de fournir tout commentaire qu'ils pouvaient avoir. Les résultats suivants ont été obtenus à partir de ces essais.

### Exemple 1 : Bonbon dur

Une formulation de bonbon dur a été préparée à partir de 120 grammes de sucre, 30 grammes d'eau, 80 grammes de sirop de glucose et 0,1 % en poids, par rapport au poids de la composition, d'arôme de cannelle (formule témoin). La formulation a été divisée en trois parties. 0,05 % en poids, par rapport au poids de la composition, de monomenthyl succinate a été ajouté à l'une des formulations de bonbon dur, et 0,05 % en poids, par rapport au poids de la composition d'une composition rafraîchissante disponible commercialement (WS3^{tm} de Wilkinson-Sword) a été ajoutée à l'autre formulation de bonbon dur aux fins de comparaison.

Le jury a trouvé que la formulation de bonbon dur contenant le monomenthyl succinate fournissait un effet rafraîchissant plaisant, de longue durée, tandis qu'aucun effet rafraîchissant n'était observé dans le témoin. Aucune saveur de menthe n'a été remarquée par le jury de dégustation. La formulation de bonbon dur contenant le WS3^{tm} a également fourni un effet rafraîchissant, bien que le jury de dégustation ait conclu que l'effet rafraîchissant du WS3^{tm} se manifestait dans une région différente de la bouche et de la gorge que l'effet rafraîchissant du monomenthyl succinate selon l'invention.

### Exemple 2 : Comprimés pressés

Du dextrose Royal T^{tm} (sucre pour compression) a été comprimé en deux comprimés, l'un contenant simplement du dextrose (témoin) et l'autre contenant 0,1 % en poids de monomenthyl succinate. Le jury de dégustation a remarqué un effet rafraîchissant plaisant pour le comprimé contenant le monomenthyl succinate et aucun effet rafraîchissant pour le comprimé témoin à base de dextrose.

### Exemple 3 : Produit de rinçage dentaire

Quatre échantillons d'une formulation de produit de rinçage dentaire ayant la composition suivante ont été testés:

| | |
|---|---|
| Chlorure de cétyl pyridinium | 0.757 grammes |
| Saccharinate de sodium | 1.75 grammes |
| Tween^{tm} 80 | 0.5 fluid ozs (1.48 E-05 m³) |
| D&C Vert#5 (solution à 0.1 % dansle propylène glycol) | 0.8 fluid ozs (2.36 E-05 m³) |
| D&C Jaune # 10 (solution à 0.1 % dansle propylène glycol) | 0.2 fluid ozs (0.59 E-05 m³) |
| Arôme N&A Mint Blend (solution à 0.2 %) | 0.25 fluid ozs (0.74 E-05 m³) |
| Alcool spécialement dénaturé | 31.0 fluid ozs (91.67 E-05 m³) |

Ces ingrédients ont été mélangés jusqu'à dissolution et ensuite 96 fluid ozs (283.87 E - 05 m³) d'eau ont été ajoutés pour fournir une formulation de rinçage de bouche. Les composés rafraîchissants ont alors été ajoutés à cette formulation dans les quantités spécifiées ci-dessous.

Les quatre formulations de rinçage de bouche testées étaient les suivantes : une ne contenait pas d'additif (témoin), une autre contenait .004 % en poids de monomenthyl succinate, une autre contenait .004 % en poids de WS3^{tm}, et la dernière contenait .002 % en poids de monomenthyl succinate et .002 % en poids de WS3^{tm}. Le jury de dégustation a noté un effet rafraîchissant dans les trois compositions, bien que le jury ait conclu que chacun des quatre échantillons de produits de rinçage dentaire présentaient un goût différent et que l'effet rafraîchissant du monomenthyl succinate se manifestait dans une région différente de la bouche et de la gorge que l'effet rafraîchissant du WS3^{tm} et du produit de rinçage. Les effets rafraîchissants du monomenthyl succinate de l'arôme de menthe du produit de rinçage et du WS3^{tm} ont été trouvés être complémentaires dans le quatrième échantillon qui contenait les trois produits rafraîchissants.

### Exemple 4 : Produit de rinçage dentaire contenant du monomenthyl succinate de sodium

Les quatre mêmes formulations de rinçage dentaire telles que préparées à l'exemple 3 ont été testées du point de vue du goût, à l'exception du fait que le monomenthyl succinate de sodium avait été substitué au monomenthyl succinate, dans les mêmes quantités. Le jury de dégustation a trouvé que les produits de rinçage de bouche contenant le monomenthyl succinate de sodium présentaient une sensation rafraîchissante de longue durée, plaisante, sans amertume. Le jury de dégustation a également noté que la sensation de rafraîchissement du monomenthyl succinate de sodium était perçue à un point légèrement en avant du point dans la bouche et dans la gorge où la sensation rafraîchissante du monomenthyl succinate avait été perçue dans les tests de goût effectués à l'exemple 3. Le monomenthyl succinate de sodium a également été jugé comme possédant un effet rafraîchissant complémentaire quand il était combiné avec le produit WS3^{tm}.

### Exemple 5 : Boisson carbonatée

Quatre échantillons chacun de boisson carbonatée à base de citron et de citron vert ont été préparées, l'un sans additif (témoin), un autre avec .004 % en poids de monomenthyl succinate, un autre avec .004 % en poids de WS3^{tm} et le dernier avec .002 % en poids de monomenthyl succinate et .002 % en poids de WS3^{tm}. La formulation de boisson carbonatée était la suivante, les parties étant exprimées en parties en poids :

| | |
|---|---|
| Sirop de glucose à haute teneur en fructose | 96.00 parties |
| Eau | 28.90 parties |
| Benzoate de sodium (solution à 25% poids/poids dans l'eau) | 0.50 parties |
| Acide citrique (solution à 50% poids/poids dans l'eau) | 2.30 parties |
| Arôme N&A citron-citron vert | 0.30 parties |

La boisson carbonatée a été formulée en combinant les trois premiers ingrédients et en les mélangeant vigoureusement, en ajoutant la solution d'acide citrique et en mélangeant de nouveau, puis en ajoutant l'arôme afin d'obtenir le sirop pour boisson. Une partie de ce sirop pour boisson a alors été combinée avec cinq parties d'eau carbonatée afin de produire la boisson carbonatée. Les composés rafraîchissants ont alors été mélangés dans les boissons carbonatées dans les quantités spécifiées ci-dessus.

Le jury de dégustation a conclu que les échantillons contenant le monomenthyl succinate et le WS3^{tm} fournissaient un effet rafraîchissant et que l'effet rafraîchissant du monomenthyl succinate se manifestait dans une région différente de la bouche et de la gorge que l'effet rafraîchissant du WS3^{tm}. De plus, l'effet rafraîchissant du monomenthyl succinate a été trouvé plaisant, sans goût de menthe, et de longue durée. En outre, la combinaison de monomenthyl succinate avec le WS3^{tm} a été jugée fournir un effet rafraîchissant complémentaire, puisque différentes régions de la bouche et de la gorge ont été affectées par les produits rafraîchissants respectifs.

### Exemples 6-8 : Boissons alcoolisées

Trois boissons alcoolisées différentes ont été préparées en utilisant 17.00 grammes de sirop de glucose à haute teneur en fructose, 140.89 grammes d'eau et 21.05, 42.11 et 84.22 grammes d'alcool de titre 190 de façon à obtenir des boissons alcoolisées de titre en alcool égal respectivement à 20,40 et 80. Aux échantillons de chacune des trois boissons alcoolisées a été ajouté 0.07 % en poids de monomenthyl succinate. Un effet rafraîchissant a été noté dans les boissons ayant un titre en alcool de 20 et 40 contenant le monomenthyl succinate mais pas dans la boisson ayant un titre de 80.

De ce fait, un nouvel échantillon de boisson de titre en alcool égal à 80 a été préparé, en utilisant une concentration plus élevée de monomenthyl succinate et, à ce niveau de plus haute concentration, un effet rafraîchissant plaisant, et de longue durée a été observé par le jury de dégustation. De plus, le jury de dégustation a conclu que le monomenthyl succinate augmentait la sensation gustative de l'alcool dans toutes les boissons alcooliques et, de ce fait, les boissons alcooliques de titre 20, 40 et 80 incluant du monomenthyl succinate étaient perçues chacune au niveau du goût comme si leur contenu en alcool était plus élevé que la boisson témoin correspondante ayant la même teneur en alcool mais pas de composé rafraîchissant.

### Exemple 9 : Chewing-gum

Une gomme base conventionnelle a été utilisée pour cet exemple. A cette base de chewing-gum a été ajouté 1.0 % en poids d'arôme de mélange de fruits rouges. Ensuite, à l'un des échantillons de chewing-gum a été ajouté 0.30 % en poids de monomenthyl succinate. Le jury de dégustation a remarqué dans le chewing-gum contenant du monomenthyl succinate un effet rafraîchissant agréable, de longue durée, à l'arrière de la bouche et de la gorge. De plus, aucun goût de menthe n'était perceptible.

### Exemple 10 : Bonbon sans sucre

Des bonbons coulés sans sucre à base de sorbitol ont été préparés en chauffant un kilo de sirop de sorbitol à 170°C, en refroidissant le sirop de sorbitol à 135°C, en ajoutant 12 grammes d'acide citrique tout en agitant et en refroidissant cette composition jusqu'à 110°C. A cet instant, 20 grammes de poudre de sorbitol et l'aromatisant incluant 0.05 % en poids, par rapport au poids de la composition, de monomenthyl succinate, ont été ajoutés à la formulation de bonbon sans sucre tout en mélangeant lentement. Finalement, les bonbons ont été coulés et laissés durcir.

Le jury de dégustation a trouvé que la formulation de bonbon dur sans sucre contenant du monomenthyl succinate fournissait un effet rafraîchissant agréable et de longue durée.

### Exemples 11-14 et exemples comparatifs A-B

Utilisation du monomenthyl succinate dans les filtres de cigarettes, en combinaison avec le menthol dans le tabac.

Les joncs de tabac de quelques cigarettes internationales standard et de cigarettes connues sous le nom de "flue-cured" ont reçu chacun une injection de 20 µl d'une solution à 10 % de cristaux de menthol dans l'éthanol.Les cigarettes dans lesquelles a été injecté le menthol ont alors été divisées en cinq lots. 20 µl, 30 µl, 40 µl et 50 µl d'une solution à 1 % de monomenthyl succinate dans la triacétine ont été injectés dans les filtres des quatre premiers lots de cigarettes, le cinquième lot étant utilisé comme témoin. Chaque lot de cigarettes comprenait un témoin ne comportant ni menthol ni monomenthyl succinate, une cigarette incluant seulement du menthol, une cigarette incluant seulement du monomenthyl succinate, et une cigarette incluant à la fois du menthol et du monomenthyl succinate.

Un panel de fumeurs experts (PFE) ont allumé, fumé et testé chaque lot des cigarettes d'essai et ont trouvé qu'à un taux de 0.2 µl de monomenthyl succinate et en l'absence de menthol, il n'y avait pas d'effet rafraîchissant. A 0.3 µl de monomenthyl succinate et en l'absence de menthol, un effet rafraîchissant léger a été noté. A un taux de 0.4 µl de monomenthyl succinate, un effet rafraîchissant élevé avec un arrière-goût rafraîchissant a été noté par le PFE. A un taux de 0.5 µl de monomenthyl succinate, l'effet rafraîchissant a encore paru plus évident au PFE et aucune augmentation de la note parfumée de la menthe du menthol n'a été notée. Cependant, une augmentation mineure de l'irritation a été observée par le PFE à ce niveau de monomenthyl succinate.

Le PFE a conclu que le monomenthyl succinate injecté dans le filtre des cigarettes avait un effet rafraîchissant sur la fumée et procurait un caractère rafraîchissant persistant dans la bouche sans altérer de façon significative la note aromatisante du menthol dans le tabac.

### Exemple 15 : utilisation de monomenthyl succinate avec l'agent rafraîchissant WS3^{tm} dans les filtres et du menthol dans le jonc de tabac

Les joncs de tabac de cigarettes choisies comme dans les exemples 1 à 4 ci-dessus ont reçu une injection de 20 µl d'une solution à 10 % de cristaux de menthol dans l'éthanol. Les cigarettes ayant subi une injection de menthol ont été divisées en deux lots. 45 µl d'une solution à 1 % de monomenthyl succinate dans la triacétine et 15 µl d'une solution à 1 % de WS3^{tm} dans la triacétine ont été injectés dans le filtre d'un lot des cigarettes contenant le menthol.

Le PFE a observé un effet rafraîchissant intense et une légère amélioration des notes de parfum aromatique et de menthe du menthol, en comparaison du témoin contenant le menthol. Aucune augmentation de l'irritation n'a été observée. Le panel a conclu qu'à ces taux d'utilisation, un effet synergique existait entre les deux agents rafraîchissants.

### Exemples 16-17

### Le monomenthyl succinate dans le tabac à mâcher et le tabac à priser

0.08 % et 0.12 % en poids de monomenthyl succinate ont été ajoutés, par injection, à du tabac à mâcher en utilisant une seringue de chromatographie à gaz de 10 µl et les mêmes niveaux de monomenthyl succinate ont été ajoutés à du tabac à chiquer, par atomisation en utilisant un atomiseur mince.

Chaque échantillon de tabac a été gardé dans la bouche des membres du PFE pour une période de test d'une minute, après quoi la bouche a été nettoyée avec de l'eau pure et une période d'attente de cinq minutes a été observée avant de recommencer une nouvelle évaluation.

A un taux de 0.08 % en poids, un effet rafraîchissant a été observé par le PFE après une période de 2-3 minutes. A 0.12 % en poids, une intensité encore plus forte de rafraîchissement a été observée approximativement deux minutes après que le tabac ait été retiré de la bouche, et l'intérieur de la bouche a été jugé comme étant plus propre. Egalement, le PFE a conclu qu'un arrière-goût rafraîchissant plus agréable était présent. L'arrière-goût a duré jusqu'à cinq minutes suivant l'élimination du tabac de la bouche.

### Exemples 18-19 : Utilisation du monomenthyl succinate dans le papier des bouts filtre des cigarettes indonésiennes

10 µl et 20 µl d'une solution à 1 % de monomenthyl succinate dans la triacétine ont été distribués sous la forme d'un film mince appliqué avec une brosse souple sur le premier tiers de l'extrémité destinée à être en contact avec la bouche, du papier pour bout filtre de différents types de cigarettes "kretek" obtenues du marché indonésien.

A 0.10 µl de monomenthyl succinate, le PFE a observé un effet légèrement rafraîchissant sur les lèvres après 2-3 minutes suivant l'insertion de la cigarette kretek dans la bouche. A 20 µl, un effet refroidissant associé avec un caractère rafraîchissant a commencé approximativement une minute après l'insertion de la cigarette kretek dans la bouche et a duré jusqu'à cinq minutes sur les lèvres et dans la partie intérieure de la bouche. Le PFE a conclu que le monomenthyl succinate avait un effet qui pouvait être efficace pour retenir un caractère propre et rafraîchissant sur les lèvres et dans la cavité intérieure de la bouche, quand il est appliqué sur l'extrémité destinée à entrer en contact avec la bouche d'une cigarette kretek.

Ces exemples ont été donnés aux seules fins d'illustration et de description et ne peuvent être interprétés comme limitant la portée de l'invention de quelque manière que ce soit. La portée de l'invention doit être déterminée d'après les revendications ci-après annexées.

## Revendications

1. Composition sélectionnée parmi les produits topiques pour humains et animaux, les produits de soin oral, les produits de soin nasal, les articles de toilette, les papiers combustibles et les feuilles d'enrobage pour produits de tabac à fumer ainsi que les filtres et les manchettes de filtres destinés à entrer en contact avec les lèvres du fumeur, les produits de tabac à mâcher, les produits de tabac à chiquer, les produits de tabac à priser, et le chewing-gum, qui comprend un produit de base et une quantité effective d'un agent rafraîchissant choisi parmi le monomenthyl succinate, les sels de métaux alcalins du monomenthyl succinate, les sels de métaux alcalino-terreux du monomenthyl succinace, et leurs mélanges.

2. Composition selon la revendication 1, dans laquelle les produits sont choisis parmi les produits topiques pour humains et animaux, les produits de soin oral, les produits de soin nasal et les articles de toilette.

3. Composition selon la revendication 1, dans laquelle les produits sont choisis parmi les produits de tabac à mâcher, les produits de tabac à chiquer et les produits de tabac à priser, et les filtres des produits de tabac à fumer.

4. Produit ingestible pour les humains et les animaux, choisi dans le groupe des articles de boulangerie, des produits laitiers, des glaces de fruits, des produits de confiserie, des confitures, des gelées, des gélatines, des puddings, des aliments pour animaux, des pastilles, des mélanges contre la toux, des décongestionnants, des antiirritants, des antacides, des préparations anti-indigestion, des analgésiques oraux, des comprimés de confiserie pressés, des bonbons durs de sucres cuits, des chewing-gums, des bonbons à base de pectine, des bonbons à mâcher, des bonbons fourrés, des fondants, des pâtes dentifrice, des produits de rinçage de la bouche, des rafraîchissants d'haleine, des boissons carbonatées, des eaux minérales, des mélanges en poudre pour boissons, des boissons non-alcooliques, des boissons alcooliques et des boissons distillées, qui comprend un produit de base et une quantité efficace d'un agent rafraîchissant sélectionné dans le groupe du monomenthyl succinate, des sels de métaux alcalins du monomenthyl succinate, des sels de métaux alcalino-terreux du monomenthyl succinate et de leurs mélanges, à condition que quand ledit produit ingestible est choisi dans le groupe des boissons alcooliques et des boissons distillées, la quantité d'agent rafraîchissant est de 0.001 % à 1.0 % en poids, par rapport au poids total du produit ingestible.

5. Produit selon la revendication 4, dans lequel le produit ingestible est un aliment choisi parmi les produits de pâtisserie, les produits laitiers, les glaces de fruits, les produits de confiserie, les bonbons et confiseries sans sucre, les confitures, les gelées, les produits à base de gélatine, les puddings et les produits alimentaires pour animaux.

6. Produit selon la revendication 4, dans lequel le produit ingestible est choisi parmi les pastilles, les mélanges contre la toux, les décongestionnants, les antiirritants, les antacides, les préparation anti-indigestion et les analgésiques oraux.

7. Produit selon la revendication 4, dans lequel le produit ingestible est choisi parmi les comprimés de confiserie pressés, les bonbons durs aux sucres cuits, les chewing-gums, les bonbons à base de pectine, les bonbons à mâcher, les fondants, les bonbons durs aux sucres cuits sans sucre, les bonbons à base de pectine sans sucre, les bonbons à mâcher sans sucre, les bonbons fourrés sans sucre, les pâtes dentifrice, les produits de rinçage de la bouche, les rafraîchissants d'haleine, les boissons alcooliques, les boissons carbonatées, les eau minérales, les mélanges en poudre pour boissons, les boissons nonalcooliques et les boissons distillées.

8. Composition ou produit selon l'une quelconque des revendications 1 à 7, comprenant de 0.001 à 1.0% en poids d'agent rafraîchissant par rapport au poids total du produit.

9. Composition aromatisante comprenant de 1 à 80 % en poids d'un agent rafraîchissant choisi dans le groupe du monomenthyl succinate, des sels de métaux alcalins du monomenthyl succinate, des sels alcalino-terreux du monomenthyl succinate et leurs mélanges, et 20 à 99 % en poids d'un diluant aromatisant qui comprend au moins un agent aromatisant choisi dans le groupe des arômes de fruits, des huiles d'herbes, des sirops sucrants ou édulcorants, des sirops aromatisants et des huiles aromatisantes, ladite composition aromatisante étant adaptée pour une utilisation dans les compositions ou les produits visés dans les revendications 1 à 8.

10. Composition aromatisante comprenant de 1 à 80% en poids d'un agent rafraîchissant choisi dans le groupe du monomenthyl succinate, des sels de métaux alcalins du monomenthyl succinate, des sels alcalino-terreux du monomenthyl succinate et leurs mélanges, et 20 à 99 % en poids d'un diluant aromatisant qui comprend au moins un agent aromatisant choisi dans le groupe des arômes de fruits, des sirops sucrants ou édulcorants, des sirops aromatisants et des huiles d'eucalyptus, de menthe poivrée, et de menthe verte.

11. Composition aromatisante comprenant de 5 à 80 % en poids d'un agent rafraîchissant choisi dans le groupe du monomenthyl succinate, des sels de métaux alcalins du monomenthyl succinate, des sels alcalino-terreux du monomenthyl succinate et leurs mélanges, et 20 à 95 % en poids d'un diluant aromatisant qui comprend au moins un agent aromatisant choisi dans le groupe des arômes de fruits, des huiles d'herbes, des sirops sucrants ou édulcorants, des sirops aromatisants et des huiles aromatisantes.

12. Composition aromatisante selon quelconque des revendications 9 à 11, comprenant de 5 à 50% en poids d'agent rafraîchissant et de 50 à 95 % de diluant aromatisant.

13. Composition aromatisante selon l'une quelconque des revendications 9 à 12, dans laquelle le diluant aromatisant comprend en outre un solvant polaire choisi parmi l'éthanol, l'acétate d'éthyle, le propylène glycol, l'alcool isopropylique et la glycérine.

14. Composition aromatisante selon l'une quelconque des revendications 9 à 13, qui comprend en outre au moins un composé rafraîchissant secondaire.

15. Composition rafraîchissante comprenant une quantité efficace d'au moins un agent rafraîchissant primaire choisi dans le groupe du monomenthyl succinate, des sels de métaux alcalins du monomenthyl succinate et des sels de métaux alcalino-terreux du monomenthyl succinate, et au moins un composé rafraîchissant secondaire.

16. Composition selon l'une ou l'autre des revendications 14 et 15, dans laquelle le composé rafraîchissant secondaire est choisi dans le groupe du menthol, des carboxamides, des cétals, de l'acétate de menthyle, du lactate de menthyle, du 3-menthoxypropane-1,2 diol et de leurs mélanges.

17. Composition rafraîchissante selon la revendication 16, dans laquelle le composé rafraîchissant à base de carboxamide est choisi parmi les produits répondant à la formule 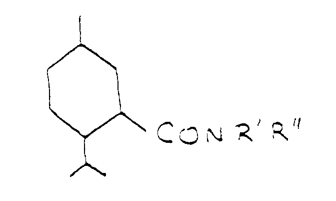 dans laquelle R', quand il est pris séparément, est l'hydrogène ou un radical aliphatique contenant jusqu'à 25 atomes de carbone ; R' ' quand il est pris séparément est un groupe hydroxy, un radical aliphatique contenant jusqu'à 25 atomes de carbone, à la condition que quand R' est l'hydrogène, R'' peut également être un radical aryle ayant jusqu'à 10 atomes de carbone et choisi parmi les groupes phényle substitué, phénalkyle, phénalkyle substitué, naphtyle, naphtyle substitué et pyrydyle ; et R' et R'', quand ils sont pris ensemble avec l'atome d'azote auquel ils sont attachés, représentent un groupe cyclique ou hétérocyclique ayant jusqu'à 25 atomes de carbone ; les carboxamides acycliques tertiaires et secondaires répondant à la formule : 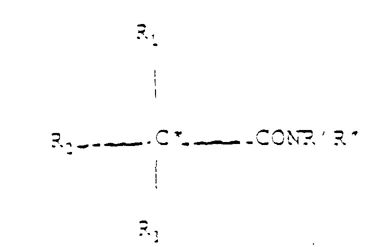 dans laquelle R' et R' ' , quand ils sont pris séparément, sont chacun l'hydrogène, un groupe alkyle en C₁-C₅ ou un groupe hydroxyalkyle en C₁-C₈ et procurent un total ce pas plus de 8 atomes de carbone, à la condition que quand R' est l'hydrogène, R'' peut également être un radical alkycarboxyalkyle ayant jusqu'à 6 atomes de carbone ; R' et R'', quand ils sont pris ensemble, représentent un groupe alkylène ayant jusqu'à 6 atomes de carbone, les extrémités opposées duquel groupe sont attachées à l'atome d'azote de l'amide afin de former un hétérocycle azoté, la chaîne carbonée duquel peut éventuellement être interrompue par l'oxygène ; R₁ est l'hydrogène ou un groupe alkyle en C₁-C₅ ; et R₂ et R₃ sont chacun un alkyle en C₁-C₅ ; avec les conditions que (i) R₁, R₂, et R₃, fournissent ensemble un total d'au moins 5 atomes de carbone, (ii) quand R₁ est l'hydrogène, R₂ est un groupe alkyle en C₂-C₅ et R₃ est un groupe alkyle en C₂-C₅ et au moins l'un des radicaux R₂ et R₃ est branché, et leurs mélanges.

18. Composition rafraîchissante selon la revendication 16, dans laquelle le composé rafraîchissant à base de cétal est choisi parmi les produits répondant à la formule : 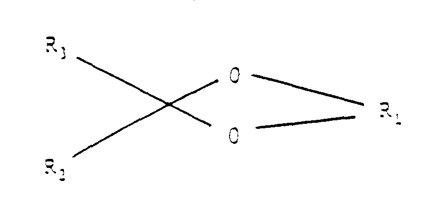 dans laquelle R₁ représence un radical alkylène en C₂-C₆ ayant au moins un, mais pas plus de trois, groupe(s) hydroxyle, et soie R₂ et R₃ indépendamment l'un de l'autre représentent un groupe alkyle en C₁-C₁₀ qui est optionnellement substitué par un à trois radicaux choisis notamment parmi les groupes hydroxyle, amino, halogène, cycloalkyle en C₅-C₇ ; aryle en C₆-C₁₂, avec 1a condition que le total des atomes de carbone de R₂ et R₃ n'est pas moins que 3, ou R₂ et R₃ ensemble représentent un radical alkylène qui, ensemble avec l'atome de carbone qui porte les radicaux R₂ et R₃, forment un noyau à 5-7 atomes, éventuellement substitué par des groupes alkyle en C₁-C₆.

## Patentansprüche

1. Zusammensetzung, die aus den folgenden Produkten ausgewählt ist: topische Produkte für Menschen und Tiere, Produkte zur Mundpflege, Produkte zur Nasenpflege, Toiletteartikel, brennbare Papiere und Umhüllungsblätter für Rauchtabakprodukte sowie Filter und Filtermanschetten, die dazu bestimmt sind, mit den Lippen des Rauchers in Kontakt zu kommen, Kautabakprodukte, Schnupftabakprodukte und Kaugummi, und die aus einem Basisprodukt und einer wirksamen Menge eines erfrischenden Mittels besteht, das aus Monomenthylsuccinat, den Alkalimetallsalzen von Monomenthylsuccinat, den Erdalkalimetallsalzen von Monomenthylsuccinat und ihren Mischungen ausgewählt ist.

2. Zusammensetzung nach Anspruch 1, bei der die Produkte aus topischen Produkten für Menschen und Tiere, Produkten zur Mundpflege, Produkten zur Nasenpflege und Toiletteartikeln ausgewählt sind.

3. Zusammensetzung nach Anspruch 1, bei der die Produkte aus Kautabakprodukten, Schnupftabakprodukten und Filtern von Rauchtabakprodukten ausgewählt sind.

4. Einnehmbare Produkte für Menschen und Tiere, die aus der Gruppe der folgenden Produkte ausgewählt sind: Backwaren, Molkereiprodukte, Fruchteis, Süßwaren, Konfitüren, Gelees, Gelatinen, Puddings, Nahrungsmittel für Tiere, Pastillen, Hustenmischungen, abschwellende Mittel, reizlindernde Mittel, Antazida, Präparate gegen Verdauungsstörungen, Oralanalgetika, gepresste Süßwarentabletten, harte Bonbons aus gekochten Zuckern, Kaugummis, Bonbons auf Pectinbasis, Kaubonbons, gefüllte Bonbons, Fondants, Zahnpasten, Produkte für die Mundspülung, Atemerfrischungsmittel, kohlensaure Getränke, Mineralwasser, Pulvermischungen für Getränke, alkoholfreie Getränke, alkoholische Getränke und destillierte Getränke, und die ein Basisprodukt und eine wirksame Menge eines erfrischenden Mittels enthalten, das aus der Gruppe ausgewählt ist, die aus Monomenthylsuccinat, den Alkalimetallsalzen von Monomenthylsuccinat, den Erdalkalimetallsalzen von Monomenthylsuccinat und ihren Mischungen besteht, sofern die Menge an erfri- schendem Mittel, wenn das einnehmbare Produkt aus der aus alkoholischen Getränken und destillierten Getränken bestehenden Gruppe ausgewählt ist, 0,001 bis 1,0 Gew.-%, bezogen auf das Gesamtgewicht des einnehmbaren Produkts, beträgt.

5. Produkt nach Anspruch 4, bei dem das einnehmbare Produkt ein Nahrungsmittel ist, das aus Konditoreiprodukten, Molkereiprodukten, Fruchteis, Süßwarenprodukten, zuckerfreien Bonbons und Süßwaren, Konfitüren, Gelees, Produkten auf Gelatinebasis, Puddings und Nahrungsmittelprodukten für Tiere ausgewählt ist.

6. Produkt nach Anspruch 4, bei dem das einnehmbare Produkt aus Pastillen, Hustenmischungen, abschwellenden Mitteln, reizlindernden Mitteln, Antazida, Präparaten gegen Verdauungsstörungen und Oralanalgetika ausgewählt ist.

7. Produkt nach Anspruch 4, bei dem das einnehmbare Produkt aus den folgenden Produkten ausgewählt ist: gepresste Süßwarentabletten, harte Bonbons aus gekochten Zuckern, Kaugummis, Bonbons auf Pectinbasis, Kaubonbons, Fondants, zuckerfreie harte Bonbons aus gekochten Zuckern, zuckerfreie Bonbons auf Pectinbasis, zuckerfreie Kaubonbons, zuckerfreie gefüllte Bonbons, Zahnpasten, Produkte für die Mundspülung, Atemerfrischungsmittel, alkoholische Getränke, kohlensaure Getränke, Mineralwasser, Pulvermischungen für Getränke, alkoholfreie Getränke und destillierte Getränke.

8. Zusammensetzung oder Produkt nach einem der Ansprüche 1 bis 7, die bzw. das 0,001 bis 1,0 Gew.-% erfrischendes Mittel, bezogen auf das Gesamtgewicht des Produkts, enthält.

9. Aromatisierende Zusammensetzung, die 1 bis 80 Gew.-% eines erfrischenden Mittels, das aus der aus Monomenthylsuccinat, den Alkalimetallsalzen von Monomenthylsuccinat, den Erdalkalimetallsalzen von Monomenthylsuccinat und ihren Mischungen bestehenden Gruppe ausgewählt ist, und 20 bis 99 Gew.-% eines aromatisierenden Verdünnungsmittels enthält, das mindestens ein Aromatisierungsmittel enthält, das aus der aus Fruchtaromen, Gewürzölen, Zucker- oder Süßungssirups, aromatisierenden Sirups und aromatisierenden Ölen bestehenden Gruppe ausgewählt ist, wobei diese aromatisierende Zusammensetzung für eine Verwendung in den Zusammensetzungen oder Produkten nach den Ansprüche 1 bis 8 angepasst ist.

10. Aromatisierende Zusammensetzung, die 1 bis 80 Gew.-% eines erfrischenden Mittels, das aus der aus Monomenthylsuccinat, den Alkalimetallsalzen von Monomenthylsuccinat, den Erdalkalimetallsalzen von Monomenthylsuccinat und ihren Mischungen bestehenden Gruppe ausgewählt ist, und 20 bis 99 Gew.-% eines aromatisierenden Verdünnungsmittels enthält, das mindestens ein Aromatisierungsmittel enthält, das aus der aus Fruchtaromen, Zucker- oder Süßungssirups, aromatisierenden Sirups und Ölen von Eukalyptus, Pfefferminze und grüner Minze bestehenden Gruppe ausgewählt ist.

11. Aromatisierende Zusammensetzung, die 5 bis 80 Gew.-% eines erfrischenden Mittels, das aus der aus Monomenthylsuccinat, den Alkalimetallsalzen von Monomenthylsuccinat, den Erdalkalimetallsalzen von Monomenthylsuccinat und ihren Mischungen bestehenden Gruppe ausgewählt ist, und 20 bis 95 Gew.-% eines aromatisierenden Verdünnungsmittels enthält, das mindestens ein Aromatisierungsmittel enthält, das aus der aus Fruchtaromen, Gewürzölen, Zucker- oder Süßungssirups, aromatisierenden Sirups und aromatisierenden Ölen bestehenden Gruppe ausgewählt ist.

12. Aromatisierende Zusammensetzung nach einem der Ansprüche 9 bis 11, die 5 bis 50 Gew.-% erfrischendes Mittel und 50 bis 95 % aromatisierendes Verdünnungsmittel enthält.

13. Aromatisierende Zusammensetzung nach einem der Ansprüche 9 bis 12, bei der das aromatisierende Verdünnungsmittel außerdem ein polares Lösungsmittel enthält, das aus Ethanol, Ethylacetat, Propylenglykol, Isopropylalkohol und Glycerin ausgewählt ist.

14. Aromatisierende Zusammensetzung nach einem der Ansprüche 9 bis 13, die außerdem mindestens eine sekundäre erfrischenden Verbindung enthält.

15. Erfrischende Zusammensetzung, die eine wirksame Menge mindestens eines primären erfrischenden Mittels, das aus der aus Monomenthylsuccinat, den Alkalimetallsalzen von Monomenthylsuccinat und den Erdalkalimetallsalzen von Monomenthylsuccinat bestehenden Gruppe ausgewählt ist, und mindestens eine sekundäre erfrischende Verbindung enthält.

16. Zusammensetzung nach einem der Ansprüche 14 und 15, bei der die sekundäre erfrischende Verbindung aus der aus Menthol, Carboxamiden, Ketalen, Menthylacetat, Menthyllactat, 3-Menthoxypropan-1,2-diol und ihren Mischungen bestehenden Gruppe ausgewählt ist.

17. Erfrischende Zusammensetzung nach Anspruch 16, bei der die erfrischende Verbindung auf Carboxamidbasis aus den folgenden Produkten ausgewählt ist: Produkte, die der Formel 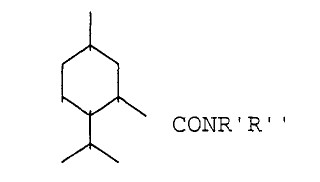 entsprechen, in der R', wenn es getrennt genommen wird, Wasserstoff oder ein aliphatischer Rest mit bis zu 25 Kohlenstoffatomen ist; R'', wenn es getrennt genommen wird, eine Hydroxygruppe, ein aliphatischer Rest mit bis zu 25 Kohlenstoffatomen ist, unter der Voraussetzung, dass, wenn R' Wasserstoff ist, R'' auch ein Arylrest mit bis zu 10 Kohlenstoffatomen sein kann, der ausgewählt ist aus den Gruppen substuiertes Phenyl, Phenalkyl, substituiertes Phenalkyl, Naphtyl, substituiertes Naphtyl und Pyrydyl; und R' und R'', wenn sie zusammen mit dem Stickstoffatom genommen werden, an das sie angelagert sind, eine zyklische oder heterozyklische Gruppe mit bis zu 25 Kohlenstoffatomen darstellen; azyklische tertiäre und sekundäre Carboxamide, die der Formel 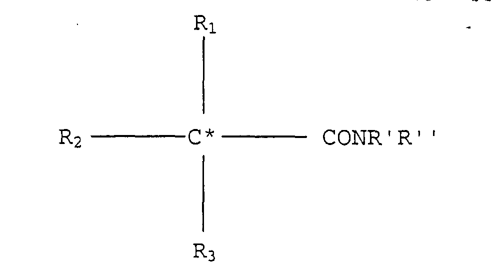 entsprechen, in der R' und R'', wenn sie getrennt genommen werden, jeweils Wasserstoff, eine Alkylgruppe mit C₁-C₅ oder eine Hydroxyalkylgruppe mit C₁-C₈ sind und insgesamt nicht mehr als 8 Kohlenstoffatome liefern, unter der Voraussetzung, dass, wenn R' Wasserstoff ist, R'' auch ein Alkycarboxyalkylrest mit bis zu 6 Kohlenstoffatomen sein kann; R' und R'', wenn sie zusammen genommen werden, eine Alkylengruppe mit bis zu 6 Kohlenstoffatomen darstellen, wobei die entgegengesetzten Enden dieser Gruppe an das Stickstoffatom des Amids angelagert sind, um einen Stickstoffheterozyklus zu bilden, dessen Kohlenstoffkette ggf. durch Sauerstoff unterbrochen sein kann; R₁ Wasserstoff oder eine Alkylgruppe mit C₁-C₅ ist; und R₂ und R₃ jeweils ein Alkyl mit C₁-C₅ ist; mit den Bedingungen, dass (i) R₁, R₂ und R₃ zusammen insgesamt mindestens 5 Kohlenstoffatome liefern, (ii) wenn R₁ Wasserstoff ist, R₂ eine Alkylgruppe mit C₂-C₅ und R₃ eine Alkylgruppe mit C₂-C₅ ist und mindestens eine der Gruppen R₂ und R₃ verzweigt ist; und ihre Mischungen.

18. Erfrischende Zusammensetzung nach Anspruch 16, bei der die erfrischende Verbindung auf Ketal-Basis aus den Produkten ausgewählt ist, die der Formel 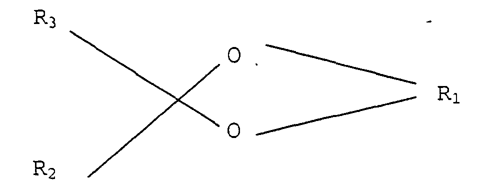 entsprechen, in der R₁ einen Alkylenrest mit C₂-C₆ darstellt, der mindestens eine, jedoch nicht mehr als drei Hydroxylgruppen hat, und entweder R₂ und R₃ unabhängig voneinander eine Alkylgruppe mit C₁-C₁₀ darstellen, die optionsweise durch ein bis drei Reste substituiert ist, die insbesondere aus den Gruppen Hydroxyl, Amin, Halogen, Cycloalkyl mit C₅-C₇, Aryl mit C₆-C₁₂ ausgewählt sind, und zwar mit der Bedingung, dass die Summe der Kohlenstoffatome von R₂ und R₃ nicht weniger als 3 ist, oder R₂ und R₃ zusammen einen Alkylenrest darstellen, der zusammen mit dem Kohlenstoffatom, das die Reste R₂ und R₃ trägt, einen Ring mit 5 bis 7 Atomen bildet, und zwar ggf. substituiert durch Alkylgruppen mit C₁-C₆.

## Claims

1. A composition selected from topical products for humans and animals, oral care products, nasal care products, toilet articles, combustible papers and leaf wrappers for smoking tobacco products, as well as filters and filter sleeves intended to come into contact with a smoker's lips, chewing tobacco products, snuff tobacco products and chewing gum, which comprises a product base and an effective amount of a coolant selected from the group comprising monomenthyl succinate, alkali metal salts of monomenthyl succinate, alkaline earth metal salts of monomenthyl succinate and mixtures thereof.

2. A composition according to claim 1, wherein the products are selected from topical products for humans and animals, oral care products, nasal care products, and toilet articles.

3. A composition according to claim 1, wherein the products are selected from chewing tobacco products, moist snuff tobacco products and snuff tobacco products, and the filters of smoking tobacco products.

4. An ingestible product for humans and animals which is selected from the group comprising baked goods, dairy products, fruit ices, confectionery products, jams, jellies, gelatins, puddings, animal feeds, lozenges, cough mixtures, decongestants, anti-irritants, antacids, anti-indigestion preparations, oral analgesics, pressed confectionery tablets, hard boiled candies, chewing gums, pectin-based candies, chewy candies, cream-centered candies, fondants, toothpastes, mouthwashes, breath fresheners, carbonated beverages, mineral waters, powdered beverage mixes, non-alcoholic beverages, alcoholic beverages and distilled beverages, which comprises a product base and an effective amount of a coolant selected from the group comprising monomenthyl succinate, alkali metal salts of monomenthyl succinate, alkaline earth metal salts of monomenthyl succinate and mixtures thereof, with the proviso that when said ingestible product is selected from the group of alcoholic beverages and distilled beverages, the coolant is present in an amount from 0.001 to 1.0% by weight, based on the total weight of the ingestible product.

5. A product according to claim 4, wherein the ingestible product is a foodstuff selected from baked goods, dairy products, fruit ices, confectionery products, sugarless candies, jams, jellies, gelatins, puddings and animal feeds.

6. A product according to claim 4, wherein the ingestible product is selected from lozenges, cough mixtures, decongestants, anti-irritants, antacids, anti-indigestion preparations, and oral analgesics.

7. A product according to claim 4, wherein the ingestible product is selected from pressed confectionery tablets, hard boiled candies, chewing gums, pectin-based candies, chewy candies, cream-centered candies, fondants, sugarless hard-boiled candies, sugarless pectin-based candies, sugarless chewy candies, sugarless cream-centered candies, toothpastes, mouthwashes, breath fresheners, alcoholic beverages, carbonated beverages, mineral waters, powdered beverage mixes, non-alcoholic beverages and distilled beverages.

8. A composition according to any one of claims 1 to 7, which comprises from 0.001 to 1.0% by weight, based on the total weight of the product, of said coolant.

9. A flavoring composition which comprises from 1 to 80% by weight of a coolant selected from the group comprising monomenthyl succinate, alkali metal salts of monomenthyl succinate, alkaline earth metal salts of monomenthyl succinate and mixtures thereof and 20-99% by weight of a flavoring diluent which comprises at least one flavoring agent selected from the group of fruit flavors, herbal oils, sweetening syrups, flavoring syrups and flavoring oils, said flavoring composition being adapted for use in the compositions or the products of claims 1 to 8.

10. A flavoring composition comprising from 1-80% by weight of a coolant selected from the group consisting of monomenthyl succinate, alkali metal salts of monomenthyl succinate, alkaline earth metal salts of monomenthyl succinate and mixtures thereof and 20-99% by weight of a flavoring diluent which comprises at least one flavoring agent selected from the group of fruit flavors, sweetening syrups, flavoring syrups and eucalyptus oils, peppermint and green mint.

11. A flavoring composition comprising from 5 to 80% by weight of a coolant selected from the group consisting of monomenthyl succinate, alkali metal salts of monomenthyl succinate, alkaline earth metal salts of monomenthyl succinate and mixtures thereof and 20-95% by weight of a flavoring diluent which comprises at least one flavoring agent selected from the group of fruit flavors, herbal oils, sweetening syrups, flavoring syrups and flavoring oils.

12. A flavoring composition according to any one of claims 9 to 11, comprising 5 to 50% by weight of the coolant and 50 to 95% of the flavoring diluent.

13. A flavoring composition according to any one of claims 9 to 12, wherein the flavoring diluent further comprises a polar solvent selected among ethanol, ethyl acetate, propylene glycol, isopropyl alcohol and glycerine.

14. A flavoring composition according to any one of claims 9 to 13, which further comprises at least one secondary coolant compound.

15. A flavoring composition comprising an effective amount of at least one primary coolant selected from the group of monomenthyl succinate and alkaline earth metal salts of monomenthyl succinate, and at least one secondary coolant compound.

16. A composition according to either one of claims 14 and 15, wherein the secondary coolant compound is selected from the group of menthol, carboxamides, ketals, menthyl actetate, menthyl lactate, 3-menthoxypropane-1,2 diol and their mixtures.

17. A coolant compostion according to claim 16, wherein the carboxamide based coolant is selected among products having the formula 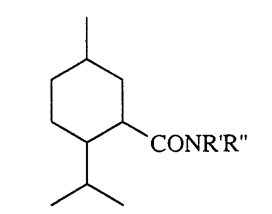 wherein R', when taken separately, is hydrogen or an aliphatic radical containing up to 25 carbon atoms; R", when taken separately, is a hydroxy group, an aliphatic radical containing up to 25 carbon atoms, with the proviso that when R' is hydrogen, R" may also be an aryl radical of up to 10 carbon atoms and selected from the group of substituted phenyl, phenalkyl, substituted phenalkyl, naphthyl, substituted naphthyl and pyridyl; and R' and R", when taken together with the nitrogen atom to which they are attached, represent a cyclic or heterocyclic group of up to 25 carbon atoms;
acyclic tertiary and secondary carboxamides of the formula: 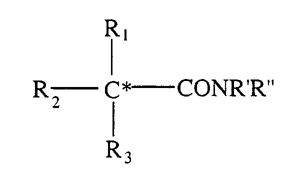 wherein R' and R", taken separately, are each hydrogen, C1-C5 alkyl or C1-C8 hydroxyalkyl, and provide a total of no more than 8 carbon atoms, with the proviso that when R' is hydrogen, R" may also be alkylcarboxyalkyl of up to 6 carbon atoms; R' and R" when taken together, represent an alkylene group of up to 6 carbon atoms, the opposite ends of which group are attched to the amide nitrogen atom so as to form a nitrogen heterocycle, the carbon chain of which may optionally be interrupted by oxygen; R1 is hydrogen or C1-C5 alkyl; and R2 and R3 are each C1-C5 alkyl; with the provisos that (i) R1, R2 and R3 together provide a total of at least 5 carbon atoms; and (ii) when R1 is hydrogen, R2 is C2-C5 lakyl and R3 is C2-C5 alkyl and at least one of R2 and R3 is branched, and mixtures thereof.

18. A coolant composition according to claim 16, wherein the ketal-based coolant is selected from the products having the formula: 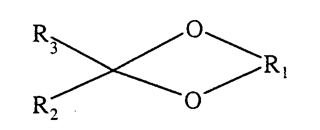 wherein R1 represents a C2-C6-alkylene radical having at least one, but not more than three, hydroxyl groups, and either R2 and R3 independently of one another represent C1-C10-alkyl which is optionally substituted by one to three radicals selected in particular among the groups hydroxyl, amino, halogen, C5-C7-cycloalkyl, C6-C12-aryl, with the proviso that the total number of carbon atoms of R2 and R3 is not less than three, or R2 and R3 together represent an alkylene radical which, together with the carbon atom which carries the R2 and R3 radicals, forms a 5-7 membered ring, optionally substituted by C1-C6-alkyl groups.
